# EUROPEAN PATENT APPLICATION

(11) **EP 4 268 740 A1**
(43) Date of publication of application: **01.11.2023**
(21) Application number: 23170114.5
(22) Date of filing: 26.04.2023
(51) Int. Cl.: A61B 17/72, A61B 17/74, A61B 17/80, A61B 17/86

(54) **PLUNGING/LINKAGE SCREW**

(30) Priority: 26.04.2022 US 202263334883 P
(71) Applicant: Stryker European Operations Limited, Carrigtwohill, Co. Cork T45 HX08 (IE)
(72) Inventor: ZANDER, Nils, 24340 Eckernförde (DE)
(74) Representative: Regimbeau

(57) **Abstract**

A method for securing a fractured bone includes inserting an intramedullary nail into an intramedullary canal of the bone; driving a bone screw into the bone such that a head of the bone screw is anchored in a cortical region of the bone; installing a bone plate against the bone to cover the head of the bone screw with an inner surface of the bone plate; and driving a plate screw through a plate hole of the bone plate into the underlying bone.

## Description

### CROSS-REFERENCE TO RELATED APPLICATIONS

The present application claims the benefit of the priority of U.S. Provisional Application No. 63/334,883 filed April 26, 2022, the disclosure of which is hereby incorporated by reference herein in its entirety.

### BACKGROUND OF THE INVENTION

The present disclosure generally relates to a fracture fixation system and a surgical method for implanting same, and more particularly, to a fracture fixation system including a bone plate, an intramedullary rod, and fasteners.

Femoral fractures are often treated with fixation devices, such as intramedullary nails or bone plates that affix the fractured bone portions together and provide stability to the bone during osteogenesis. Surgical methods are known for reconstructing a patient's anatomy after a bone fracture. These procedures rely on the operator's use of fasteners which are driven to a fractured bone in order to reset comminuted areas of the fractured bone. The bone plates may carry the load of the comminuted area of the bone. Due to the rigidity of the plate minimizing the motion of the fractured bone, the fractured bone can heal in the proper position.

In the case of intramedullary nails, a surgeon inserts a nail into the intramedullary canal of a patient and then inserts a lag screw or barrel through the intramedullary nail and into the neck of the femur to prevent relative movement of the fractured bone portions. Bone plates, often used in conjunction with intramedullary nails, are usually contoured to the surface shape of the bone. Typically, an operator will drive screws through the bone plate and then through transverse bores of the intramedullary nail.

Traditional intramedullary nails and bone plating systems are not without drawbacks. For example, due to the specific locations at which they must be inserted, bone screws that are driven through a bone plate and through the intramedullary nail cannot be anchored in multiple portions of cortical bone where improved fixation strength is found. Further, traditional bone plating systems may only allow bone screws to be driven into the underlying bone at specific angles that are not advantageous to a patient's unique fractures.

Thus, a fracture fixation system that allows for greater autonomy with regard to the insertion of fasteners is desired.

### BRIEF SUMMARY OF THE INVENTION

In one aspect, a method for securing a fractured bone comprises inserting an intramedullary nail into an intramedullary canal of the bone; driving a bone screw into the bone such that a head of the bone screw is anchored in a cortical region of the bone; installing a bone plate against the bone such that an inner surface of the bone plate at least partially covers the head of the bone screw within a plane perpendicular to a central axis of the bone screw; and driving a plate screw through a plate hole of the bone plate into the underlying bone.

In another aspect, the step of driving the plate screw includes driving the plate screw through the plate hole into a first hole of the intramedullary nail.

In a further aspect, the step of driving the plate screw includes driving the plate screw through the plate hole in a direction passing alongside a longitudinal axis of the intramedullary nail.

In another aspect, the step of driving the bone screw includes anchoring a tip of the bone screw in an opposed cortical region of the bone.

In another aspect, the step of driving the bone screw includes driving the bone screw into the bone such that a top surface of the head of the bone screw is flush with an outer surface of the bone.

In a different aspect, the step of driving the bone screw includes driving the bone screw into the bone such that a top surface of the head of the bone screw is sunk beneath an outer surface of the bone.

In a further aspect, the step of driving the bone screw includes driving the bone screw into a second hole of the intramedullary nail.

In another aspect, the method further comprises drilling a pilot hole into the bone and through the second hole of the intramedullary nail.

In another aspect, the method further comprises drilling a countersunk recess concentric with the pilot hole in the cortical region of the bone.

In a different aspect, the step of driving the bone screw includes driving a shank of the bone screw into the pilot hole and the head of the bone screw into the countersunk recess.

In another aspect, the step of driving the bone screw includes driving the bone screw so that it does not contact or pass through a hole of the intramedullary nail.

In a further aspect, the method further comprises driving a second bone screw into a third hole of the intramedullary nail such that a top surface of the head of the second bone screw is flush with the outer surface of the bone.

In another aspect, the method further comprises driving a second plate screw through a second plate hole of the bone plate and into a fourth hole of the intramedullary nail.

In a different aspect, a bone fracture fixation system comprises an intramedullary nail; a bone plate including a plate hole extending therethrough; and a bone screw comprising a shank, a head, and threads disposed on the shank and the head, the head of the bone screw being adjacent or contacting an inner surface of the plate.

In another aspect, the inner surface of the bone plate at least partially covers the head of the bone screw within a plane perpendicular to a central axis of the bone screw.

In a further aspect, the inner surface of the bone plate fully covers the head of the bone screw within a plane perpendicular to a central axis of the bone screw.

In another aspect, the threads of the bone screw comprise a uniform thread pitch and direction across the shank and the head.

In yet another aspect, the shank of the bone screw has a uniform diameter.

In a different aspect, a top surface of the head of the bone screw is flush with a surface of the bone in the implanted configuration.

In another aspect, the bone plate does not contact the head of the bone screw.

In a further aspect, the head of the bone screw sits flush with a surface of the bone in the implanted configuration.

In a different aspect, a portion of the inner surface of the bone plate adjacent or contacting the head of the bone screw is devoid of any screw holes.

In another aspect, the inner surface is substantially smooth.

In a further aspect, the bone screw extends through a second hole of the intramedullary nail.

In another aspect, the first and second holes of the intramedullary nail define respective axes that are angularly offset from each other.

### BRIEF DESCRIPTION OF THE DRAWINGS

The features, aspects, and advantages of the present invention will become better understood with regard to the following description, appended claims, and accompanying drawings in which:
FIG. 1 is a perspective view from a side angle of a fracture fixation system.
FIG. 2 is a perspective side view of a plunger screw of the fracture fixation system of FIG. 1.
FIG. 3 is a perspective side view of a hybrid screw of the fracture fixation system of FIG. 1.
FIG. 4 is another perspective view from a side angle of the fracture fixation system of FIG. 1 including the plunger screw of FIG. 2 and the hybrid screw of FIG. 3.
FIG. 5 is a perspective view of another embodiment of a fracture fixation system.
FIG. 6 is a perspective view of yet another embodiment of the fracture fixation system of FIG. 1.
FIG. 7 is a side view of a drill sleeve used to implant the fracture fixation system of FIG. 1 and the corresponding hole structures created with the drill sleeve.

### DETAILED DESCRIPTION OF THE PREFERRED EMBODIMENTS

As used herein, when referring to bones or other parts of the body, the term "proximal" means closer to an operator and the term "distal" means further away from the operator. The term "anterior" means toward the front of the patient's body and the term "posterior" means toward the back of the patient's body. The term "inferior" means toward the feet and the term "superior" means towards the head. The term "medial" means toward the midline of the body and the term "lateral" means away from the midline of the body.

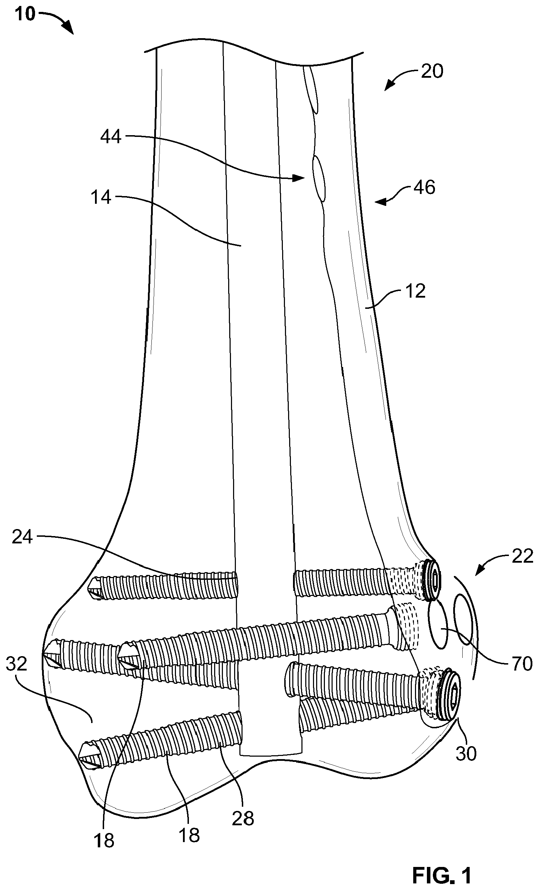

FIG. 1 illustrates a perspective view of fracture fixation system 10 for securing a fractured femur. Although the embodiments depicted herein describe and illustrate a femur, it is envisioned that the fracture fixation system 10 may be compatible with other bones, such as the tibia, humerus, and other long bones. Fracture fixation system 10 includes an elongate bone plate 12 and an elongate intramedullary nail 14. A plurality of linking fasteners 16 extend from the bone plate through the intramedullary nail 14. A plurality of unlinked fasteners 18 extend through intramedullary nail 14 without extending through bone plate 12.

Intramedullary nail 14 defines a longitudinal axis and extends along that axis from a superior end 20 to an inferior end 22. Each of superior end 20 and inferior end 20 corresponds to either a leading end or a trailing end depending on the insertion location of the intramedullary nail. Intramedullary nail 14 preferably has a circular cross-section and is inserted into the medullary canal of the patient's bone, which contains marrow and is circumferentially encased by cortex which provides a dense outer surface that fasteners can engage with.

One or more holes 24 are defined by and extend transversely through intramedullary nail 14. The diameter of holes 24 may correspond to the diameter of linked or unlinked fasteners that will be driven therethrough. Accordingly, the diameters of holes 24 may vary from one hole to the next depending on factors such as the patient's bone structure or the size of the fastener required. Each hole 24 defines an axis extending therethrough. Each hole axis may be parallel to the other hole axes or may be angularly offset therefrom. For example, the first four superior holes 24 in FIG. 1 have parallel hole axes to orient fasteners extending therethrough towards bone plate 12. The three inferior holes each have hole axes extending at different angles relative to the four superior hole axes. These axes allow the corresponding fasteners to extend towards the anterior and posterior ends of bone plate 12 without contacting bone plate 12. Holes 24 may have an internal thread pattern that corresponds to the thread pattern of fasteners configured to extend therethrough. Different hole arrangements are envisioned depending on several factors, such as the geometry of the patient's fracture and the quantity and size of fasteners required to secure that fracture.

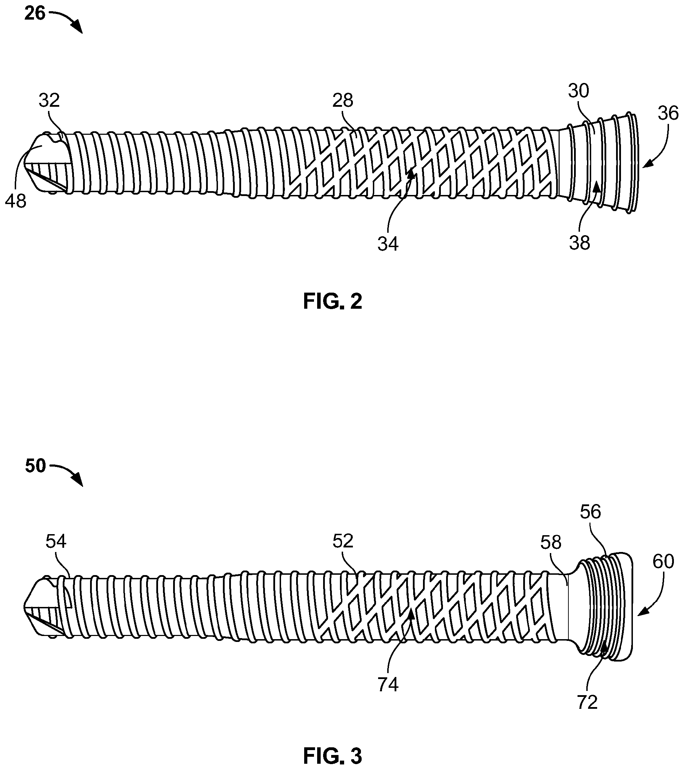

FIG. 2 illustrates an unlinked fastener according to a preferred embodiment of a bone screw 26, also referred to as a plunger screw. Bone screw 26 comprises a shank 28, a screw head 30 at a proximal end, and a screw tip 32 at a distal end. Shank 28 extends along a longitudinal axis and has a generally cylindrical shape. Shank 28 preferably has a diameter the varies across shank 28, and may also have a diameter that tapers toward its distal end. Shank 28 comprises a thread pattern 34 that matches a thread pattern of the screw head 30 and the screw tip 32; that is, the pitch of the thread along the entire screw 26 is the same. Thread pattern 34 may include entirely lefthand threads, entirely righthand threads, or a superimposed righthand thread and lefthand thread. All of screw 26 or a portion thereof, such as the shank and tip, can be the same as the screws described in U.S. Pat. Pub. No. 2021/0338294, the disclosure of which is incorporated by reference herein. The superimposed thread type may be beneficial in simplifying removal of the screw if the screw breaks during insertion or under a load. The uniform thread pattern across the screw head 30, shank 28, and screw tip 32 is beneficial in that it limits the possibility of cross-threading upon insertion or removal of the plunger screw.

Screw head 30 has a generally conical taper extending from its proximal end toward its distal end. Such a taper may cause a seamless transition between screw head 30 and shank 28. A recess 36 formed in the proximal end of screw head 30 is configured to receive an insertion tool, such as a screwdriver or a wrench. This configuration allows bone screw 26 to be rotated about its longitudinal axis to be driven into bone. Recess 36 may have any shape known in the art to engage the insertion tool. For example, recess 36 could have a square or a hexagonal shape to engage with the corresponding shape of a driver. Screw head 30 has an outer thread 38 that has the same pitch as the thread pattern 34 of shank 28. Outer threads 38 are configured to engage with a cortical region of bone to provide strong fixation. Further, because of the conical taper of screw head 30, screw head 30 may be driven into a countersunk recess 40 to sit flush with a surface of the bone or below a surface of the bone. This allows inner surface 44 of bone plate 12 to be placed against the underlying bone without interfering with screw head 30. In this configuration, screw head 30 is shielded by bone plate 12 while simultaneously engaging the dense cortical bone structure.

Screw tip 32 extends from the distal end of shank 28. Screw tip 32 may taper to a point or to a blunt nose, such as a rounded nose. Screw tip 32 may comprise at least one groove 48 configured to act as a self-tapping guide as bone screw 26 is driven into bone. Groove 48 may include two diametrically opposed grooves, four grooves spaced 90° apart from each other, or another groove arrangement known in the art. Groove 48 may be at least partially helical and may define an edge that expels bone or other materials away from the insertion direction of bone screw 26. Screw tip 32 has a thread pattern that is identical to the thread pattern of screw head 30 and shank 28 to prevent the possibility of cross-threading upon insertion or removal of bone screw 26.

FIG. 3 illustrates a hybrid screw 50, also referred to as a plate screw, that is preferably utilized as a linking screw 16, but may also be utilized as an unlinked fastener 18 in alternative embodiments. The hybrid screw 50 design is substantially similar to the design of bone screw 26. Hybrid screw 50 comprises a shank 52, a screw head 56, and a screw tip 54. The shank 52 and screw tip 54 of hybrid screw 50 are substantially similar to the corresponding features of bone screw 26 described herein, while the screw head 56 differs. Screw head 56 has a generally conical taper from its proximal end to its distal end that can be configured to sit in a countersunk recess 40 in a bone. A neck portion 58 extends between screw head 56 and shank 52. Neck portion 58 is unthreaded and has a diameter that is smaller than the proximal portion of shank 52 and screw head 56. A recess 60 is included in proximal end of screw head 56. Recess 60 is shaped to receive a distal end of an insertion tool, similar to the bone screw 26. Unlike screw head 30 of bone screw 26, screw head 56 of hybrid screw 50 may have a thread pattern with a self-cutting geometry. The self-cutting geometry may include cutting flutes or other geometries known in the art. Such a self-cutting geometry aids screw head 56 in insertion into bone whether a countersunk recess is first formed in bone or not formed at all. Screw head 56 may also anchor into an aperture of the bone plate in a linked configuration.

The design of hybrid screw 50 is advantageous because it allows the screw head 56 to anchor into a bone plate or into a cortical region of the bone. When hybrid screw 50 may act as a linking screw 16 and may be driven through a hole 70 in bone plate 12 and through intramedullary nail 14. In such a configuration, screw head 56 is anchored into the contouring of hole 70 rather than a cortical region of bone while the screw tip 54 extends through the intramedullary nail 14 and into an opposing cortical region of bone or into the femoral head. Alternatively, hybrid screw 50 may act as an unlinked fastener 18 and is not driven through bone plate 12. Rather, the threaded region of screw head 56 may be driven into a countersunk recess 40. Thus, the screw head 56 is anchored in a cortical region of bone and screw tip 54 is anchored in an opposing cortical region of bone.

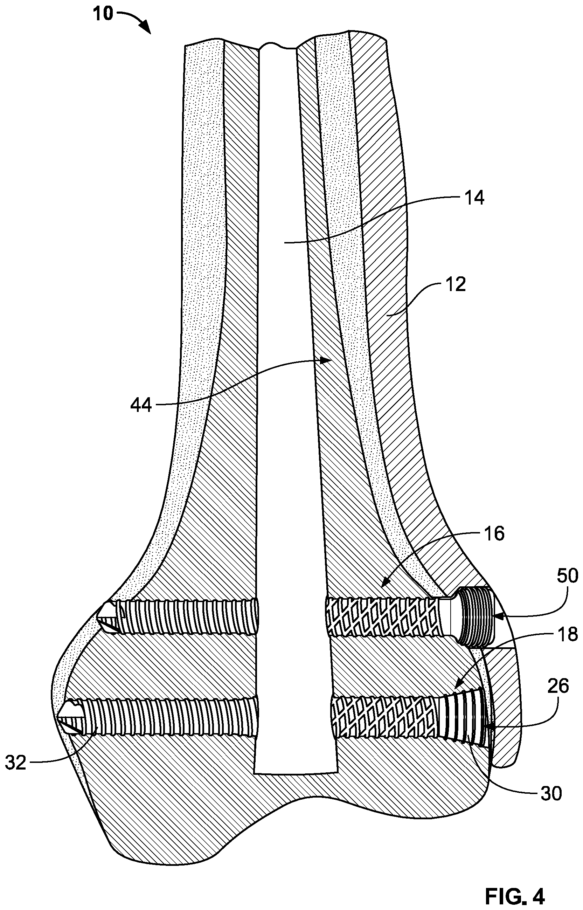

FIGS. 1 and 4 depict a bone plate 12 used with fracture fixation system 10. Bone plate 12 is elongated and extends from a superior end 20 to an inferior end 22. An inner surface 44 of bone plate 12 is configured to face the bone and an outer surface 46 of bone plate is configured to face away from the bone, e.g., in a lateral direction. Inner surface 44 may be substantially concave to contour to the underlying bone surface. The curvature of inner surface 44 may also be configured to initially define a gap between inner surface 44 and the underlying bone. Inner surface 44 may then flex such that the gap is closed when fasteners are driven though bone plate 12.

Inner surface 44 of bone plate 12 is substantially smooth and lacks slots, cutouts, or other regions (aside from any separate screw holes therethrough, such as hole 70) that could accept a head of an underlying fastener. Bone plate 12 is configured to transition between an implanted configuration and a non-implanted configuration. In an implanted configuration, the substantially smooth inner surface 44 of bone plate 12 is configured to sit flush or substantially flush with the underlying bone. Because inner surface 44 is substantially smooth, unlinked fasteners 18, such as bone screws, that are anchored into the underlying cortical bone may sit flush with the outer cortical bone surface or be sunk beneath the cortical surface to avoid interfering with the seating of the bone plate on the bone. In such a manner, the inner surface 44 of bone plate 12 may cover an aperture or a portion of an aperture in bone defined by the screw heads driven therein.

Bone plate 12 defines at least one hole 70 extending transverse to the longitudinal axis of the plate. Hole 70 is configured to receive a fastener therethrough. The fastener may be a bone screw, nail, beam, or other fastener type known in the art. Hole 70 may further have contouring along its internal surface to engage with the head of the fastener being driven therethrough. Such contouring may allow for fastener insertion at variable angles while maintaining a strong connection with the head of the fastener. The curvature of bone plate 12 and the contouring of hole 70 allows each fastener to be driven into the underlying bone at optimized angles to reach and stabilize the fragmented bone. Locking features such as washers or plates may be utilized on the outer surface 46 of bone plate 12 to prevent fasteners from backing out of the bone and bone plate.

Bone plate 12 is an elongated member formed from a single piece of rigid material, such as stainless steel, titanium, or another rigid material known in the art. The bone plate 12 may have a substantially uniform plate thickness, or it may have varying thicknesses depending on the orientation and type of fasteners extending through bone plate 12, e.g., the plate may be thicker in regions to accommodate a cross brace or support pin therethrough. Bone plate 12 may be configured to extend along an inferior region of a femur, along a superior region of a femur, or long the entire length of a femur depending upon the nature of the patient's fracture.

FIG. 4 depicts fracture fixation system 10 in conjunction with the inferior region of the femur. As shown, inner surface 44 of bone plate 12 is contoured to the underlying bone in the implanted configuration. Intramedullary nail 14 extends longitudinally through the underlying medullary canal along an axis that is roughly parallel to the plate axis. Unlinked fasteners 18, such as the bone screw 26, are configured to extend through intramedullary nail 14 but not through bone plate 12. Screw heads 30 of unlinked fasteners 18 are anchored in a cortical region of bone. Anchoring screw heads in the cortical region is advantageous because it allows an operator the autonomy to choose fastening locations without being limited by the geometry of the bone plate. Screw tip 32 may then extend into a diametrically opposed region of cortical bone or into another bone feature, such as the femoral head. Because screw head 30 of unlinked fastener 18 is not attached to bone plate 12, it is free to be implanted at any point around the circumference of the cortical bone. Thus, unlinked fasteners 18 can extend at multiple angles through intramedullary nail 14 to reach a desired fracture location.

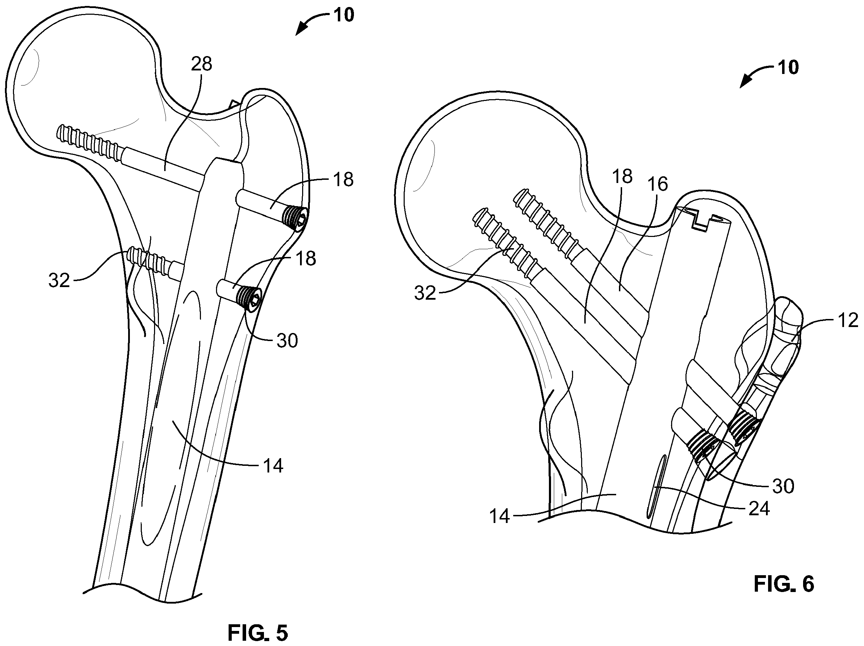

Screw head 30 of unlinked fastener 18 is configured to engage the cortical region of bone without interfering with the bone plate 10. As shown in FIG. 4, screw head 30 may sit flush with a surface of the bone but does not protrude proud with respect to the surface of the bone as it would then interfere with the implanted bone plate 12. Alternatively, screw head 30 may sit beneath the surface of the bone, which also allows the inner surface 44 of bone plate 12 to more closely match the contour of the underlying bone without interference. In such a manner, unlinked fastener 18 may extend at one of multiple angles along its longitudinal axis through intramedullary nail 14 to allow both screw head 30 and screw tip 32 to anchor in cortical bone. Further, because the screw head 30 is driven into a countersunk recess without interfering with bone plate 12, the inner surface 44 of bone plate 12 will cover the countersunk recess regardless of the angle at which the unlinked fastener 18 extends. Thus, the inner surface 44 of bone plate 12 may cover the countersunk recess 40 in a plane normal to the central longitudinal axis of the countersunk recess 40. In other words, an inner surface of bone plate 12 at least partially covers screw head 30 within a plane perpendicular to the central axis of unlinked fastener 18. In other embodiments, the inner surface of bone plate 12 fully covers screw head 30 within a plane perpendicular to the central axis of unlinked fastener 18. Ultimately, the coverage of unlinked fastener 18 over screw head 30 is such that when screw head 30 is viewed along the central longitudinal axis of unlinked fastener 18, the bone plate 12 either at least partially or fully covers screw head 30, as the case may be. In other embodiments, this coverage can be defined by the aperture in the bone created by the countersunk recess 40. That is, if unlinked fastener 18 is angled away from the central axis of the bone in which it is inserted, the bone plate 12 may at least partially or fully cover the aperture within the bone when viewed from an axis normal to the bone and passing through a center of the aperture at the countersunk recess 40. Screw tip 32 may extend to various regions of bone. In certain embodiments, such as the embodiment depicted in FIG. 5 showing a retrograde nail inserted from the knee, unlinked fastener 18 may extend from one cortical region of bone to an opposing cortical region of bone. In another embodiment depicted in FIG. 6 in which an antegrade nail is inserted from the hip, screw head 30 may be anchored in the cortical bone underneath bone plate 12 in the implanted configuration. Shank 28 extends distally from screw head 30 through intramedullary nail 14 toward the femoral head. Screw tip 32 may then anchor within the femoral head.

Linking screws 16, such as the hybrid screw 50, extend through holes 70 in bone plate 12 and through holes 24 in intramedullary nail 14. In the implanted configuration, screw head 56 engages with hole 70 of bone plate 12 and screw tip 54 passes through intramedullary nail 14 to anchor into an opposing cortical region of bone. Screw head 56 is configured to engage with the contouring of hole 70 and may extend at variable angles through bone plate 12 towards the holes 24 of intramedullary nail 14. Because holes 24 of intramedullary nail 14 have axes extending at different angles relative to each other, linked screws 16 may extend through bone at various angles relative to bone plate 12. Partially linked fasteners 76 extend from bone plate 12 into bone without passing through intramedullary nail 14. In another embodiment, linked screws 16 extend from bone plate 12 though intramedullary nail 14 and unlinked fasteners 18 extend through intramedullary nail 14 and pass alongside bone plate 12, i.e., to the anterior or posterior side of bone plate 12.

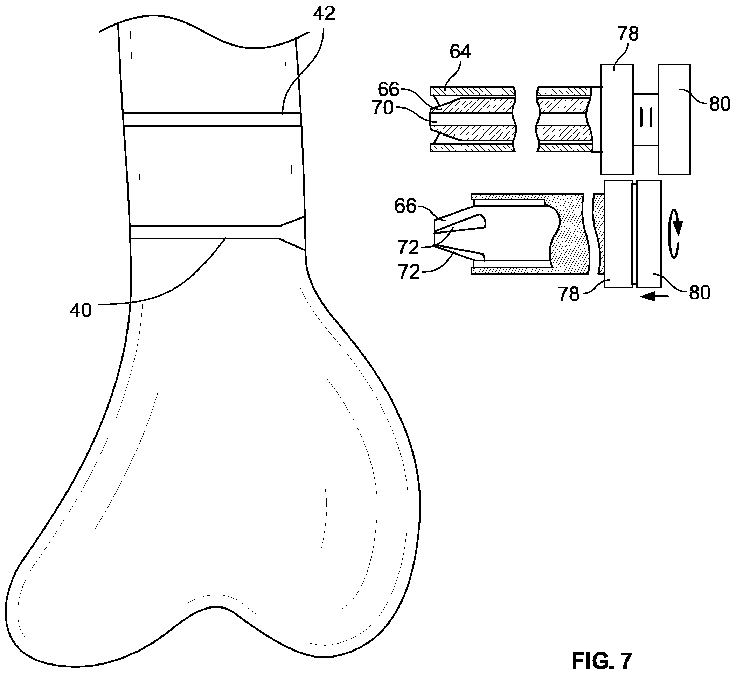

FIG. 7 depicts a drill sleeve 62 according to one embodiment. Drill sleeve 62 includes a cannula 64 configured to spread and hold tissue to allow access to the underlying bone. Guide 66 is axially translatable within cannula 64 and tapers toward its distal end. Guide 68 defines a lumen 70 concentrically located within guide 68. Drill bits or driving bits may be placed within lumen 70 for various drilling functions, such as drilling a pilot hole. The distal end of guide 66 is conical and of a larger diameter than any drill bit that fits within lumen 70, and includes at least one cutting flute 72 designed to cut into bone. Cutting flute 72 may include multiple cutting flutes spaced radially around guide 66 or it may include other flute designs known in the art. The distal end of guide 66 is conically tapered, in some embodiments to mimic the shape of the corresponding screw head 30. In such a manner, when guide 66 is rotated and advanced toward the bone, cutting flutes 72 create a countersunk hole dimensioned to receive conical screw head 30 so that screw head 30 does not interfere with bone plate 12. Guide 66 is rotatably coupled with an enlarged proximal end 80 that can be rotated either manually or with an automatic driver while holding a proximal end 78 of cannula 64 stationary. Proximal end 78 of cannula 64 and proximal end 80 of guide 66 may include knurling or other grip-enhancing textures on their outer surfaces to aid an operator in rotation.

A method of securing a fracture using fracture fixation system 10 is provided. First, an intramedullary nail 14 is inserted into a canal of a long bone using insertion methods known in the art, such as inserting a retrograde nail or an antegrade nail from the patient's knee or hip, respectively.

In a next step, a pilot hole 42 is drilled from one outer surface of cortical bone through hole 24 of the intramedullary nail to the diametrically opposed cortical bone surface. Pilot hole 42 may be drilled using drill sleeve 62. An operator may secure a drill bit in lumen 70 of guide 66 to drill the pilot holes. An operator may then engage a distal end of cannula 64 with a patient's tissue to open the tissue and allow access to the underlying bone. An operator may then rotate the proximal end 80 of guide 66 while holding proximal end 78 of cannula 64 stationary to advance guide 66 toward the bone. Manual rotation of proximal end 80 in a first direction rotates guide 66 and advances it distally to allow cutting flute 72 to begin cutting a countersunk recess 40 into bone. Once the recess 40 has been created, the operator may rotate proximal end 80 in the opposite direction to retract guide 66 distally within cannula 64. These steps may then be repeated to drill multiple pilot holes and countersunk recesses. Countersunk recess 40 is drilled to a depth that allows screw head 30 to sit flush or nearly flush with the cortical surface of bone or below the cortical surface of bone to limit interference with bone plate 12 so that the inner surface 44 of bone plate 12 defines a plane that covers countersunk recess 40 or a portion of countersunk recess 40 at a tangential point where a longitudinal axis of bone screw 26 approaches or would extend through inner surface 44. Bone screw 26 is then driven through the pilot hole so that shank 28 extends through hole 24 and screw tip 32 engages with the opposed cortical region of bone from screw head 30. Screw head 30 is driven to a depth where its outer threads engage with the cortical bone but the proximal end of the screw head is at least flush or entirely beneath the cortical bone surface to minimize interference with bone plate 12. An operator may then repeat these steps with a plurality of bone screws 26 to engage with the patient's unique fracture geometry.

The pilot holes 42 for the linking screws 16 may be drilled through the holes 70 of bone plate 12 after it has been placed on a surface of the bone to assist with alignment. Once the pilot holes 42 have been created, linking screw 16 may be driven through the hole 70 of bone plate 12 so that shank 52 extends through hole 24 of intramedullary nail 14 and screw tip 54 extends into the far cortical bone or into another portion of bone, such as the femoral head. Outer threads 72 of screw head 56 are engaged with the contouring of holes 70 to prevent the linking screw 16 from backing out of bone plate 12. An operator may then insert additional linking screws through other holes 70 of bone plate 12 and holes of intramedullary nail 14 to secure the bone plate in an implanted configuration. An operator may also drive screws through holes 70 of bone plate 12 to engage the underlying bone, but those screws do not pass through or contact intramedullary nail 14. These screws may be inserted before, after, or simultaneously with linking screws 16 depending on the specific order determined by the operator to limit the chances of the screws interfering with each other.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to a further advantage. Any reference signs in the claims should not be construed as limiting the scope.

## Claims

1. A bone fracture fixation system (10) comprising:
an intramedullary nail (14);
a bone plate (12) including a plate hole (24) extending therethrough; and
a bone screw (26) comprising a shank (28), a head (30), and threads (34) disposed on the shank (28) and the head (30), the head (30) of the bone screw (26) being adjacent or contacting an inner surface (44) of the plate (12).

2. The system (10) of claim 1, wherein an inner surface (44) of the bone plate (12) at least partially covers the head (30) of the bone screw (26) within a plane perpendicular to a central axis of the bone screw (26).

3. The system (10) of claim 1, wherein an inner surface (16) of the bone plate (12) fully covers the head (30) of the bone screw (26) within a plane perpendicular to a central axis of the bone screw (26).

4. The system (10) of claim 1, wherein the threads (34) of the bone screw (26) comprise a uniform thread pitch and direction across the shank (28) and the head (30).

5. The system (10) of claim 1, wherein the shank (28) of the bone screw (26) has a uniform diameter.

6. The system (10) of claim 1, wherein a top surface of the head (30) of the bone screw (26) is flush with an outer surface of the bone.

7. The system (10) of claim 1, wherein the bone plate (12) does not contact the head (30) of the bone screw (26).

8. The system (10) of claim 1, wherein the head (30) of the bone screw (26) sits flush with a surface of the bone in the implanted configuration.

9. The system (10) of claim 1, wherein a portion of the inner surface (44) of the bone plate (12) adjacent or contacting the head (30) of the bone screw (26) is devoid of any screw holes.

10. The system (10) of claim 1, wherein the inner surface (26) is substantially smooth.

11. The system (10) of claim 1, wherein the bone screw (26) extends through a second hole (24) of the intramedullary nail (14).

12. The system (10) of any one of claims 1-11, wherein the plate and second holes (24) of the intramedullary nail (14) define respective axes that are angularly offset from each other.

13. The system (10) of claim 1, further comprising a plate screw (50) including a shank (52), a head (56), and a tip (54).

14. The system (10) of any one of claims 1-13, wherein the head (56) of the plate screw (50) is conically tapered.

15. The system (10) of any one of claim 1-14, wherein the head (56) of the plate screw (50) is configured to anchor into a hole (70) of the bone plate (12) and the shank (52) of the plate screw (50) is configured to extend through a hole (24) of the intramedullary nail (14).
